# EUROPEAN PATENT SPECIFICATION

(11) **EP 1 566 157 B1**
(45) Date of publication and mention of the grant of the patent: **31.08.2011**
(21) Application number: 03754157.0
(22) Date of filing: 16.10.2003
(51) Int. Cl.: A61F 13/58

(54) **DISPOSABLE WEARING ARTICLE**
TRAGBARER EINWEG-ARTIKEL
ARTICLE VESTIMENTAIRE JETABLE

(30) Priority: 17.10.2002 JP 2002303498; 01.10.2003 JP 2003343595
(43) Date of publication of application: 24.08.2005
(73) Proprietor: UNI-CHARM CO., LTD., Kawanoe, Ehime 799-0111 (JP)
(72) Inventor: ITO, Kyoko c/o Technical Center, Toyohama-cho, Mitoyo-gun Kagawa 769-1602 (JP); SATO, Junko c/o Technical Center, Toyohama-cho Mitoyo-gun Kagawa 769-1602 (JP); SASAKI, Toru c/o Technical Center, Toyohama-cho Mitoyo-gun Kagawa 769-1602 (JP)
(74) Representative: Sperling, Rüdiger
(86) International application number: PCT/JP2003/013266
(87) International publication number: WO 2004/034942

(56) References cited:
- EP-A1- 0 648 483
- JP-A- 10 085 254
- JP-A- 10 085 254
- JP-A- 10 110 144
- US-A- 5 234 517

## Description

### TECHNICAL FIELD OF THE INVENTION

The present invention relates to a disposable wearing article provided with tape fasteners. The wearing article includes disposable diapers of pull-on type and open-type having tape fasteners respectively.

### BACKGROUND ART OF THE INVENTION

Japanese Utility Model Application Publication No. 1994-77719A (Reference 1) discloses a pull-on disposable diaper comprising a liquid-pervious topsheet, a liquid-impervious backsheet and an absorbent pad interposed between these two sheets wherein front and rear waist regions are joined together to form a waist-hole and a pair of leg-holes. In this pull-on diaper of well known art, a waist's intermediate region defined between the waist-hole and the leg-holes is provided with elastic members so that the intermediate region is formed with gathers as the elastic members contract in a waist-surrounding direction. The backsheet is provided on its outer surface with tape fasteners having finger-grips, respectively, so that the respective finger-grips may be oriented in the waist-surrounding direction.

### DISCLOSURE OF THE INVENTION

The tape fastener of well known art disclosed in the above-cited Publication is folded in its longitudinal direction in Z- or inverted Z-shape and maintained in this Z- or inverted Z-shape by adhesive applied on segments of a tape tab for the fastener. However, depending on the adhesive force of the adhesive, respective tape segments forming the Z- or inverted Z-shape may be peeled off one from another as the waist's intermediate region is formed with the gathers and obstruct the diaper against being smoothly put on the wearer's body.

It is an object of the present invention to provide a wearing article having tape fasteners provided with fastening means improved so that these tape fasteners may be reliably held on the article before the article is actually used and easily peeled off from the article first when the article is actually used.

According to the present invention, there is provided a disposable wearing article having a surface to contact with a wearer's body and a surface to contact with wearer's clothes, the article being contoured by front and rear end zones extending in parallel to each other in a waist-surrounding direction and a pair of opposed side edge zones extending in parallel to each other in a longitudinal direction, the article being composed, in the longitudinal direction orthogonal to the waist-surrounding direction, of a crotch region, a front waist region extending from the crotch region to the front end zone and a rear waist region extending from the crotch region to the rear end zone, the front and rear waist regions being releasably or permanently joined together along the side edge zones so as to form a waist-hole and a pair of leg-holes, the side edge zones of the front or rear waist region being provided with tape fasteners attached thereto so as to extend in the waist-surrounding direction, a tape tab forming each of the tape fasteners having a fixed end zone and a free end zone serving as a finger-grip opposed to each other in a longitudinal direction of the tape tab, the fixed end zone being permanently attached to the surface of the wearing article to contact with the wearer's clothes, the tape tab being adapted to be unfolded from the fixed end zone toward the free end zone as the tape fastener is pulled in the waist-surrounding direction with the free end zone gripped between fingers and, of an inner surface of the tape fastener facing the surface of the wearing article to contact with the wearer's clothes as the tape fastener is unfolded and a surface opposed to the inner surface, the inner surface being at least partially provided with a fastening means attachable to a specified part in any of the inner surface and the surface opposed to the inner surface.

The present invention further comprises the tape material is folded in a Z- or inverted Z-shape so that the tape tab can be unfolded from the fixed end zone toward the free end zone, the tape fastener comprising a first tape segment defining an uppermost layer of the Z- or inverted Z-shape and having the free end zone and a first end zone opposed to the free end zone in the longitudinal direction, the first tape segment being provided, between the both end zones, on the inner surface with a fastening means, a second tape segment lying between the first tape segment and the surface of the wearing article to contact with the wearer's clothes so as to face the surface of the wearing article to contact with the wearer's clothes and having the fixed end zone and a second end zone opposed to the fixed end zone, and a third tape segment interposed between the first tape segment and the second tape segment and connecting the tape segments to each other, the third tape segment having one.end zone connected to the first end zone of the first tape segment and the other end zone opposed to the one end zone thereof being connected to the second end zone of the second tape segment and an intermediate zone defined between the one end zone and the other end zone being releasably attached to the first tape segment by means of the fastening means, the one end zone extending in the waist-surrounding direction beyond the fixed end zone of the second tape segment and releasably attached to the surface of the wearing article to contact with the wearer' s clothes, and the outer surface of the tape tab folded in any of the Z- or inverted Z-shape is opposed to itself in a non-attached condition in a vicinity of a region in which the second end zone of the second tape segment and the other end zone of the third tape segment are connected to each other.

In the disposable wearing article according to the present invention the tape fastener does not unintentionally peel off from the article, because one end of the third tape segment is extending beyond the fixed end of the second tape segment and releasably attached to the article on its surface to contact with the wearer's clothes. In the vicinity of the region in which the second tape segment and the third tape segment are connected to each other, the outer surface of the tape fastener is folded back and facing itself in a non-attaching condition, so the tape fastener can be easily unfolded merely by pulling the outer end zone of the first tape segment of the tape fastener outwardly of the waist region.

The present invention includes the following embodiments.
(1) The fastening means is an adhesive coated on the inner surface of the first tape segment. This fastening means can be attached to various materials such as nonwoven fabric and plastic film used in the wearing article.
(2) The fastening means is a hook part provided on the inner surface of a mechanical fastener comprising the hook part and a loop part and the specified part is provided with the loop part. This fastening means is suitable for a use to repeat attaching and releasing of the means on the wearing article.
(3) The loop part is a nonwoven fabric to form any of the surfaces of the wearing article to contact with the wearer's skin or the wearer's clothes. In this embodiment of the present invention there is no need to prepare any specific material for the loop art of the mechanical fastener.
(4) The first end zone of the first tape segment extends in the waist-surrounding direction beyond the one end zone of the third tape segment and is releasably attached to the surface of the wearing article to contact with the wearer's clothes. In a wearing article of this embodiment the tape fastener does not easily peel off from the surface of the wearing article.
(5) The one end zone of the third tape segment extends in the waist-surrounding direction beyond the fixed end zone of the second tape segment by a dimension in a range of 1 to 15 mm.
(6) The wearing article may be any of an open-type disposable diaper and a pull-on type disposable diaper. The tape fastener of this embodiment can be used to fit the diaper around the waist of the wearer and also to roll-up the diaper after use.

### BRIEF DESCRIPTION OF THE DRAWINGS

Fig. 1 is a partially cutaway plan view showing a disposable diaper;
Fig. 2 is a sectional view taken along a line II-II in Fig. 1;
Fig. 3 is a view similar to Fig. 2 showing an initial stage of unfolding of the tape fastener;
Fig. 4 is a diagram illustrating a manner in which the tape fasteners are used;
Fig. 5 is a diagram illustrating another manner in which the tape fasteners are used;
Fig. 6 is a view similar to Fig. 2 showing one preferred embodiment of the invention; and
Fig. 7 is a view similar to Fig. 2 showing another preferred embodiment of the invention.

### DESCRIPTION OF THE BEST MODE FOR WORKING OF THE INVENTION

Details of the disposable wearing article according to the present invention will be more fully understood from the description of a disposable diaper of open-type as a specific embodiment of the invention given hereunder with reference to the accompanying drawings.

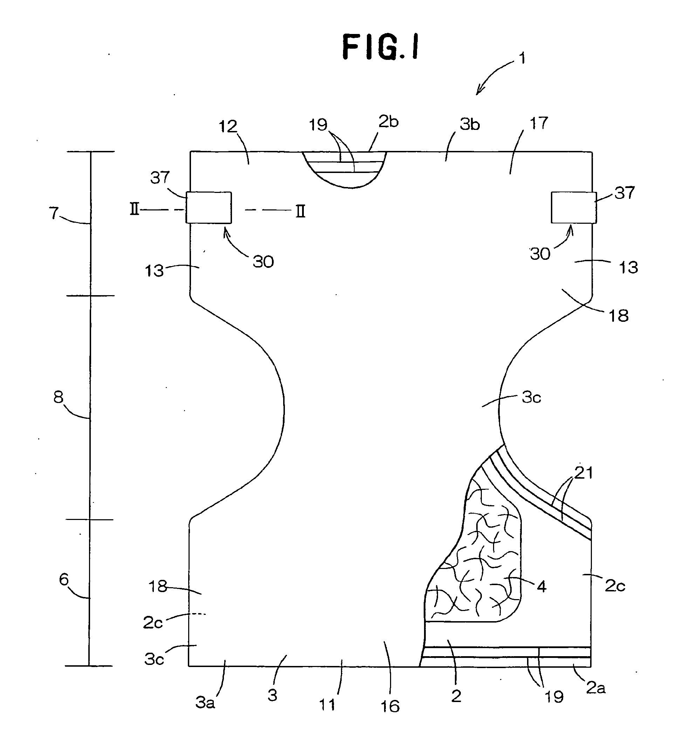

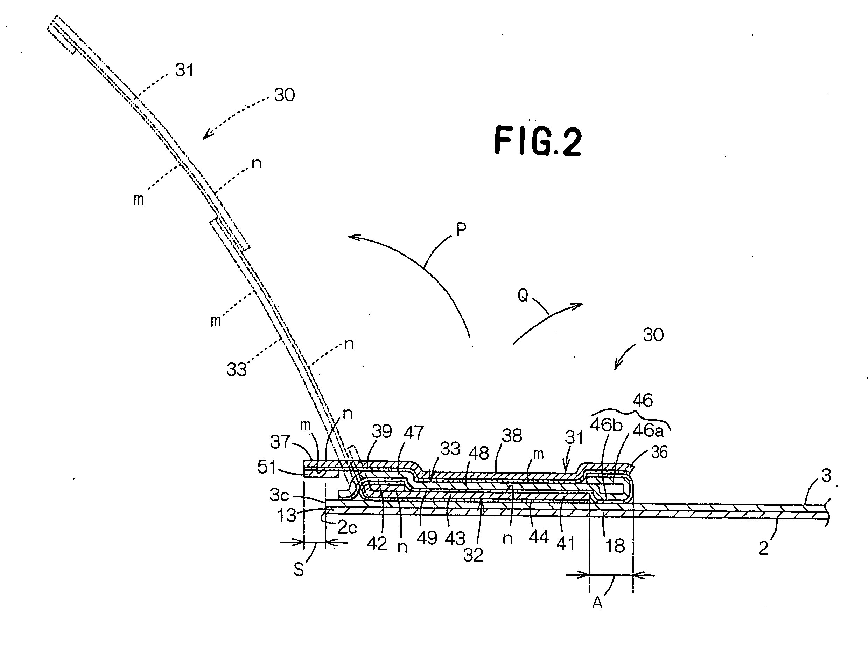

Fig. 1 is a partially cutaway plan view showing a disposable diaper 1 of open-type and Fig. 2 is a sectional view taken along a line II-II in Fig. 1. The diaper 1 basically comprises a liquid-pervious topsheet 2 defining a surface to contact with a wearer's body, a liquid-impervious backsheet 3 to contact with the wearer' s clothes and a liquid-absorbent core 4 interposed between these sheets 2, 3. In Fig. 1, the backsheet 3 is illustrated to overlie the topsheet 2. The diaper 1 is contoured by front and rear end zones 11, 12 extending in parallel to each other in a waist-circumferential direction (transverse direction as viewed in Fig. 1) and a pair of side edge zones 13 extending in parallel to each other in a longitudinal direction which is orthogonal to the waist-circumferential direction. In this longitudinal direction, the diaper 1 is composed of a crotch region 8, a front waist region 6 extending between the crotch region 8 and the front end zone 11, and a rear waist region 7 extending between the crotch region 8 and the rear end zone 12. In the crotch region 8, the side edge zones 13 are curved inward. The top- and backsheets 2, 3 extend outward beyond a peripheral edge of the core 4. These portions extending beyond the peripheral edge of the core 4 are overlaid and attached together by means of a hot melt adhesive (not shown) so as to form a front flap 16, a rear flap 17 and side flaps 18. In the front flap 16 and the rear flap 17, waist elastic members 19 are interposed between the top- and backsheets 2, 3 and bonded in a stretched condition to the inner surface of the topsheet 2 and/or the backsheet 3. In the side flaps 18, leg elastic members 21 are interposed between the top- and backsheets 2, 3 along the curved side edge zones 13 in the crotch region 8 and bonded in a stretched condition to the inner surface of the topsheet 2 and/or the backsheet 3. In the rear waist region 7, the respective side flaps 18 have tape fasteners 30 attached to the backsheet 3 in the vicinity of the respective side edge zones 13. These tape fasteners 30 have their lengths extending respectively in the waist-surrounding direction and are folded in a Z- or inverted Z-shape so that the tape fasteners 30 can be unfolded outward beyond the side edge zones 13 of the diaper 1. The top- and backsheets 2, 3 respectively have front and rear end zones 2a, 2b, 3a, 3b defining the front and rear end zones 11, 12 of the diaper 1 and side edge zones 2c, 3c defining the side edge zones 13 of the diaper 1. The topsheet 2 is formed with a liquid pervious nonwoven fabric, a liquid pervious perforated plastic film etc. The backsheet 3 is formed with a liquid impervious nonwoven fabric, a liquid impervious plastic film, a composite from a liquid impervious plastic film and a nonwoven fabric laminated thereon etc.

As will be understood from Fig. 2, each of the tape fastener 30 in a folded state comprises a top tape segment 31 defining a first tape segment, a bottom tape segment 32 defining a second tape segment and an intermediate tape segment 33 defining a third tape segment wherein each of these tape segments 31 through 33 has an inner surface m facing the backsheet 3 when the tape fastener 30 has been unfolded as indicated by imaginary lines and an outer surface n opposed to the surface m. The top tape segment 31 has an inner end zone 36 lying inside the diaper 1 as viewed in the waist-circumferential direction, an outer end zone 37 preferably including a part thereof extending outward beyond the side edge zone 13 so that this outer end zone 37 can be used as a finger-grip and an intermediate zone 38 lying inside the side edge zone 13 wherein the inside surface m of these zones 36 through 38 are coated on the lower surfaces thereof with a second adhesive 39. Referring to Fig. 2, a dimension by which the outer end zone 37 extends outward beyond the side edge zone 13 is indicated by S which is preferably 0.7 mm or longer.

The bottom tape segment 32 lying on the side edge zone 13 and facing the surface of the diaper 1 to contact with the wearer's clothes has an inner end zone 41, an outer end zone 42 and an intermediate zone 43 wherein at least the inner end zone 41 defining the fixed end zone of the tape fastener 30 is permanently attached to the backsheet 3 by means of a first adhesive 44. In the case of the preferred bottom tape segment 32, it will be understood from Fig. 2 that the inner end zone 41, the intermediate zone 43 and the outer end zone 42 except its portion folded back and connected to the intermediate tape segment 33 are permanently attached to the backsheet 3 by means of the first adhesive 44 applied on the inner surfaces m of these zones respectively. The portion of the outer end zone 42 folded back has its surface facing the intermediate tape segment 33 is permanently attached to the intermediate tape segment 33 by means of the first adhesive 44 and its outer surface n facing each other is not attached to itself.

The intermediate tape section 33 also lies inside the side edge zone 13 and has an inner end zone 46, an outer end zone 47 and an intermediate zone 48 wherein respective outer surfaces n of these zones are coated with third adhesive 46.

In such tape fastener 30, the inner end zone 36 of the top tape segment 31 is permanently attached to a portion 46a folded upward from the inner end zone 46 of the intermediate tape segment 33. The outer end zone 37 of the top tape segment 31 is coated with the second adhesive 39 over an appropriate range inclusive of a portion of the outer end zone 37 extending outward in the longitudinal direction of this top tape segment 31 beyond the side edge zone 13 by the dimension S. However, the second adhesive 39 is covered with a film strip 51 and thereby the inner and outer surfaces m, n may become substantially nonadhesive to define the free end zone serving as a finger-grip for the tape fastener 30. The intermediate zone 38 of the top tape segment 31 is releasably attached to the inner surface m of the intermediate tape segment 33 underlying the intermediate zone 38 of the top tape segment 33, i.e. the segment 33 located between the intermediate zone 38 and the bottom tape segment 32.

The distal half 46a folded upward from the inner end zone 46 extending in the waist-surrounding direction of the intermediate tape segment 33 is permanently attached to the top tape segment 31 and the proximal half 46b is releasably attached to the backsheet 3. The proximal half 46b has preferably a length A of 1 to 15mm as measured in the longitudinal direction of the intermediate tape segment 33. The outer end zone 47 of the intermediate tape segment 33 is permanently attached to the outer end zone 42 of the bottom tape segment 32. The zone of the intermediate tape segment 33 except for the inner and outer end zones 46, 47, i.e., the intermediate zone 48 of the intermediate tape segment 33 is releasably attached to the outer surface n of the bottom tape segment 32 by means of a third adhesive 49. It should be understood that it is possible without departing from the scope of the invention to releasably bond the intermediate zone 48 of the intermediate tape segment 33 to one of the top tape segment 31 and the bottom tape segment 32.

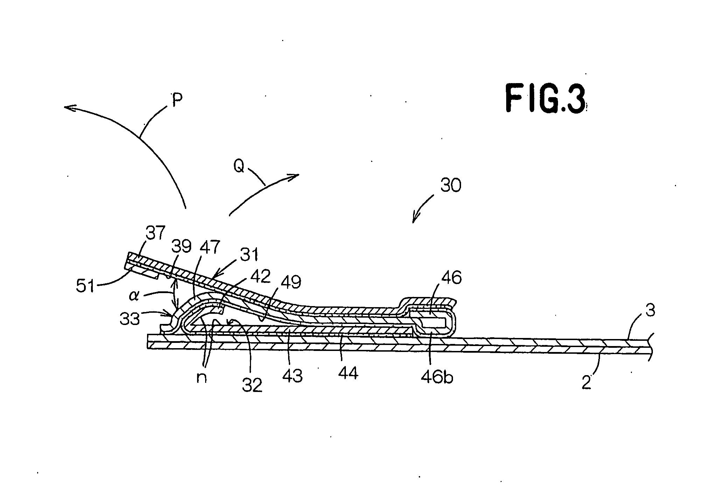

Fig. 3 is a view similar to Fig. 2 showing an initial stage of unfolding of the tape fastener 30. The tape fastener 30 comprises the top tape segment 31, the intermediate tape segment 33 and the bottom tape segment 32 contiguously connected one to another and folded in a Z-shape. Finger-gripping the outer end zone 37 of the top tape segment 31 together with the film strip 51, the tape fastener 30 may be either pulled outwardly in the waist region of the diaper 1 in a direction indicated by an arrow P or pulled inwardly in the waist region of the diaper 1 in a direction indicated by an arrow Q to unfold the tape fastener 30 as indicated by the imaginary lines in Fig. 2. More specifically, it will be understood from Fig. 3 that one of the corners forming the Z-shape at which the bottom tape segment 32 is connected to the intermediate tape segment 33 is lifted up as the tape fastener 30 is pulled in this manner because the outer surface n of the bottom tape segment 32 faces itself in a non-attaching condition in the vicinity of this corner. Consequently, the top tape segment 31 and the intermediate tape segment 33 are spaced from each other at an angle α and so-called a peel force works between these top and intermediate tape segments 31, 33. Under this peel force, these two tape segments 31, 33 are smoothly peeled off from each other. Successively, the proximal half 46b of the inner end zone 46 of the intermediate tape segment 33 is peeled off from the backsheet 3 and an intermediate zone defined between the inner end zone 46 and the outer end zone 47 of the intermediate tape segment 33 is peeled off from the intermediate zone 43 of the bottom tape segment 32. Afterward the tape fastener 30 extends in such a manner as depicted by an imaginary line of Fig. 2. The top tape segment 31 of the tape fastener 30 completely unfolded may be attached by a fastening means, i.e. the adhesive 39, to a specified part of the diaper 1. Such tape fastener 30 can be unfolded merely by pulling the fastener 30 outwardly of the side edge zone 13 and it is not essential to reverse the top tape segment 31 as indicated by the arrow Q in order to unfold the tape fastener 30. From this viewpoint, it is easy to use this tape fastener 30.

In the tape fastener 30, the attaching force of the second adhesive 39 and the third adhesive 49 can be adjusted in various manners so that the second adhesive 39 may be easily peeled off from the intermediate tape segment 33 and the third adhesive 49 may be easily peeled off from the bottom tape segment 32. Such an adjustment can be achieved, for example, by previously selecting the second adhesive 39 and the third adhesive 49 exhibiting the adhesive force lower than that exhibited by the first adhesive 44. It is also possible for such an adjustment to coat the outer surface n of these tape segments 32, 33 with an appropriate release agent such as a silicone oil.

While the tape fastener 30 can be smoothly unfolded, for example, by pulling the tape fastener 30 in the direction indicated by the arrow P when the tape fastener 30 is actually used, the proximal half 46b of the inner end zone 46 of the intermediate tape segment 33 releasably attached to the backsheet 3 neither extends nor peel off unintentionally from the backsheet 3 before an actual use of the tape fastener 30. Even if the outer surface n is entirely coated with a silicone oil, the proximal half 46b of the intermediate tape segment 33 is attached not to the surface n but to the backsheet 3 usually free from coating of a release agent such as a silicone oil. Therefore, it is not likely that the proximal half 46b of the intermediate tape segment 33 might be peeled off from the backsheet 3 unless the tape fastener 30 is pulled in the direction indicated by the arrow P or Q.

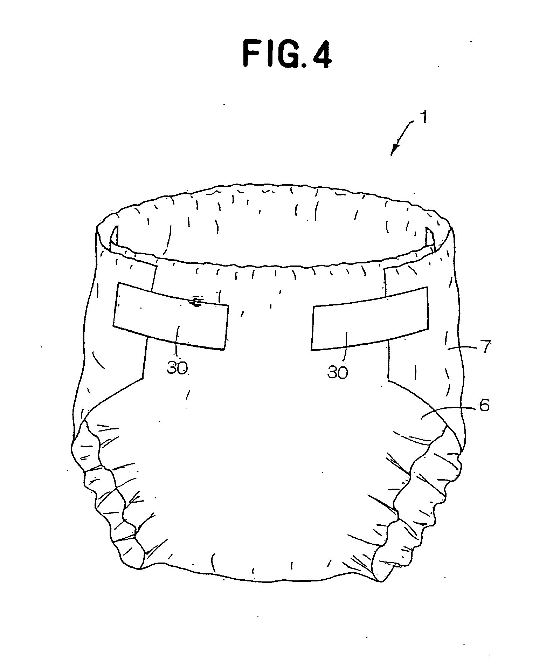

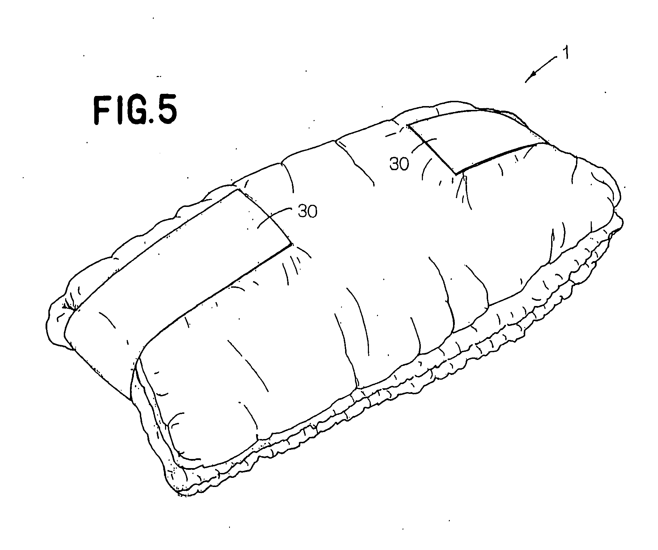

Figs. 4 and 5 are diagrams illustrating manners in which the tape fasteners 30 are used. In Fig. 4, the diaper 1 is put on the wearer' s body and the tape fasteners 30 unfolded outward from the rear waist region 7 and releasably attached to the surface of the front waist region 6 facing the wearer' s clothes (not shown) by means of the second adhesive 39. In Fig. 5, the used diaper 1 has been rolled up and is held by the tape fasteners 30 in such a rolled up state. In this way, these tape fasteners 30 are useful not only to put the diaper 1 on the wearer's body but also to hold the used diaper 1 in a rolled up state for disposal.

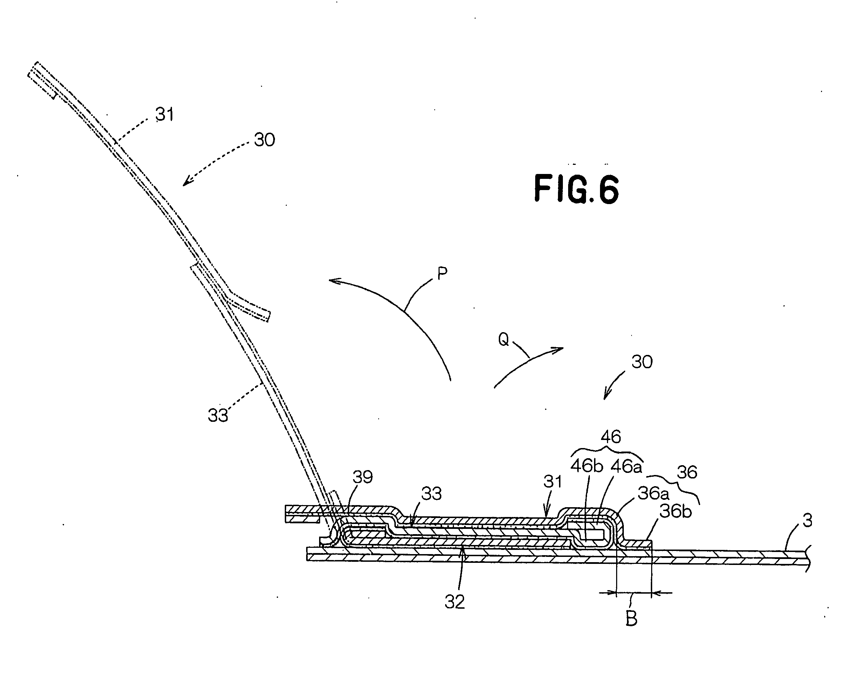

Fig. 6 is a view similar to Fig. 2 showing one preferred embodiment of the invention. In the tape fastener 30 attached to the diaper 1 according to this embodiment, the proximal half 36a partially forming the inner end zone 36 of the top tape segment 31 is permanently attached to the distal half 46a, on one hand, and the distal half 36b extends inwardly of the diaper 1 beyond the inner end zone 46 of the intermediate tape segment 33 and releasably bonded to the backsheet 3 by means of the second adhesive 39, on the other hand. The distal half 36b cooperates with the distal half 46b of the intermediate tape segment 33 to prevent the tape fastener 30 from peeling off from the diaper 1 particularly in its region releasably attached to the backsheet 3. Such preventive effect of the distal half 36b may be further improved by adjusting a dimension B by which the distal half 36b extends inwardly of the diaper 1 to approximately 5 mm. Taking account of both the embodiment shown in Fig. 2 and the embodiment shown in Fig. 6, the dimension B can be specified to be in a range of 0 to 5 mm.

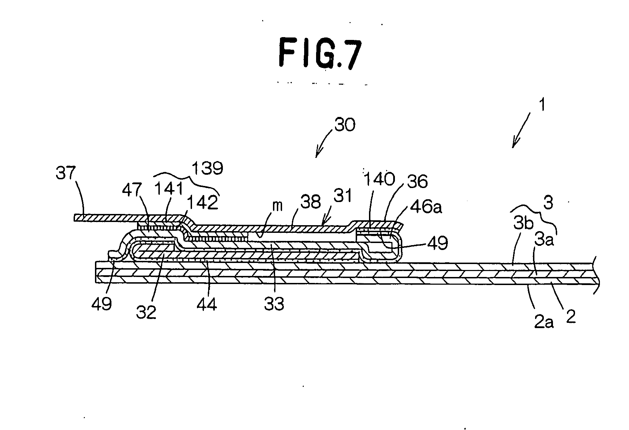

Fig. 7 is a view similar to Fig. 2 showing another preferred embodiment of the present invention. The top tape segment 31 of the tape fastener 30 is formed with a plastic film or a nonwoven fabric. The inner end zone 36 of the top tape segment 31 is coated with a forth adhesive 140 and the inner end zone 46a of the intermediate tape segment 33 is coated with the third adhesive 49. Both of the inner end zones 36, 46a are permanently attached to each other. The inner surface m of the intermediate zone 38 of the top tape segment 31 is provided with a hook part 139 of a mechanical fastener comprising the hook part and a loop part. The hook part 139 has a base sheet 141 and plural hooks 142 extending vertically to the base sheet 141. The base sheet 141 is permanently attached to the inner surface m of the intermediate zone 38. The intermediate tape segment 33 is formed with a nonwoven fabric which has plural loops releasably engageable with the hooks 142. The bottom tape segment 32 is formed with a plastic film or a nonwoven fabric. The bottom tape segment 32 is permanently attached to the backsheet 3 with the first adhesive 44 and also permanently attached to the outer end zone 47 of the intermediate tape segment 33. The topsheet of the diaper 1 is formed with a nonwoven fabric 2a and the backsheet 3 is formed with a composite sheet of a plastic film 3a and a nonwoven fabric 3b laminated on the outer surface of the plastic film 3a. These nonwoven fabrics 2a and 3b have respectively plural loops engagemable with the hooks 142. When the outer end 37 of the top tape segment 31 is pulled in the direction indicated by the arrow P of Fig. 1, the tape fastener 30 is deformed as depicted in Fig. 3 and extended straight after the hooks 142 disengage from the loops of the intermediate tape segment 33. The hooks 142 of the tape fastener 30 extended in such a manner can be engaged as a fastening means with the loops provided on any specified parts of the nonwoven fabrics 2a and/or 3b. The diaper 1 having the tape fastener 30 as depicted in Fig. 7 may have the backsheet 3 formed with a plastic film which is provided with a target zone made of a nonwoven fabric engageable with the hooks 142.

While the invention has been described hereinabove with respect to the open-type disposable diaper as a specific embodiment, the invention is applicable also to other disposable wearing articles such as a pull-on type disposable diaper prepared by permanently joining the front and rear waist regions 6, 7 of the diaper 1 together along the respective side edge zones 13 by use of an adhesive or a welding technique, a diaper for incontinent patients or a diaper for training purpose.

## Claims

1. A disposable wearing article (1) having a surface to contact with a wearer's body and a surface to contact with wearer's clothes, said article being contoured by front and rear end zones (11, 12) extending in parallel to each other in a waist-surrounding direction and a pair of opposed side edge zones (13) extending in parallel to each other in a longitudinal direction, said article being composed, in said longitudinal direction orthogonal to said waist-surrounding direction, of a crotch region (8), a front waist region (6) extending from said crotch region (8) to said front end zone (11) and a rear waist region (7) extending from said crotch region (8) to said rear end zone (12), said front and rear waist regions (6, 7) being releasably or permanently joined together along said side edge zones (13) so as to form a waist-hole and a pair of leg-holes, said side edge zones (13) of said front or rear waist region (6, 7) being provided with tape fasteners (30) attached thereto so as to extend in said waist-surrounding direction, a tape tab forming each of said tape fasteners having a fixed end zone and a free end zone serving as a finger-grip opposed to each other in a longitudinal direction of said tape tab, said fixed end zone being permanently attached to said surface of said wearing article to contact with the wearer' s clothes, said tape fastener being adapted to be unfolded from said fixed end zone toward said free end zone as said tape fastener (30) is pulled in said waist-surrounding direction with said free end zone (37) gripped between fingers and, of an inner surface of said tape fastener facing said surface of said wearing article to contact with the wearer's clothes as said tape fastener is unfolded and a surface opposed to said inner surface, said inner surface being at least partially provided with a fastening means attachable to a specified part in any of the inner surface and the surface opposed to said inner surface, said disposable wearing article further comprising:
said tape material (30) being folded in a Z- or inverted Z-shape so that said tape material can be unfolded from said fixed end zone toward said free end zone, said tape fastener comprising a first tape segment (31) defining an uppermost layer of said Z- or inverted Z-shape and having said free end zone (37) and a first end zone (36) opposed to said free end zone (37) in said longitudinal direction, said first tape segment (31) being provided, between said both end zones (36, 37), on said inner surface with said fastening means, a second tape segment (32) lying between said first tape segment (31) and said surface (3) of said wearing article (1) to contact with the wearer's clothes so as to face said surface of said wearing article to contact with said wearer's clothes and having said fixed end zone (42) and a second end zone (41) opposed to said fixed end zone (42), and a third tape segment (33) interposed between said first tape segment (31) and said second tape segment (32) and connecting said tape segments (31,32) to each other, said third tape segment (33) having one end zone (46) connected to said first end zone (36) of said first tape segment (31) and the other end zone (47) opposed to said one end zone thereof being connected to said second end zone (42) of said second tape segment (32) and an intermediate zone (48) defined between said one end zone (46) and said other end zone (47) being releasably attached to said first tape segment (31) by means of said fastening means adhesive (39), said one end zone (46) extending in said waist-surrounding direction beyond said fixed end zone (41) of said second tape segment (32) and releasably attached to said surface (3) of said wearing article (1) to contact with said wearer' s clothes, and said outer surface of said tape tab folded in any of said Z- or inverted Z-shape is opposed to itself in a non-attached condition in a vicinity of a region in which said second end zone (42) of said second tape segment (38) and said other end zone (47) of said third tape segment (33) are connected to each other.

2. The disposable wearing article according to Claim 1, wherein said fastening means (39) is an adhesive coated on said inner surface of said first tape segment (31).

3. The disposable wearing article according to Claim 1, wherein said fastening means is a hook part provided on said inner surface of a mechanical fastener comprising said hook part and a loop part and said specified part is provided with said loop part.

4. The disposable wearing article according to Claim 3, wherein said loop part is a nonwoven fabric to form any of said surfaces to contact with said wearer's skin or said wearer's clothes.

5. The disposable wearing article according to any one of Claims 1 to 4, wherein said first end zone (36) of said first tape segment (31) extends in said waist-surrounding direction beyond said one end zone (46) of said third tape segment (33) and is releasably attached to said surface (3) of said wearing article (1) to contact with said wearer's clothes.

6. The disposable wearing article according to any one of Claims 1 to 5, wherein said-one end zone (46) of said third tape segment (33) extends in said waist-surrounding direction beyond said fixed end zone (41) of said second tape segment (32) by a dimension in a range of 1 to 15 mm.

7. The disposable wearing article according to any one of Claims 1 to 6, wherein said wearing article is any of an open-type disposable diaper and a pull-on type disposable diaper.

## Patentansprüche

1. Wegwerf-Bekleidungsartikel (1), der eine Oberfläche für den Kontakt mit dem Körper des Trägers und eine Oberfläche für den Kontakt mit der Kleidung des Trägers hat, welcher Artikel durch eine vordere und eine hintere Endzone (11, 12) umrissen ist, die parallel zueinander in einer Hüftumfangsrichtung verlaufen, und ein Paar von einander gegenüberliegenden Seltenrandzonen (13), die in einer Längsrichtung parallel zuelnander verlaufen, welcher Artikel in der Längsrichtung senkrecht zu der Hüftumfangsrichtung aus einem Schrittbereich (8), einem vorderen Hüftbereich (6), der von dem Schrittbereich (8) zu der vorderen Endzone (11) verläuft, und einem hinteren Hüftbereich (7) zusammengesetzt ist, der von dem Schrittbereich (8) zu der hinteren Endzone (12) verläuft, wobei der vordere und der hintere Hüftbereich (6, 7) entlang den Seitenrandzonen (13) lösbar oder dauerhaft miteinander verbunden sind, um so eine Hüftöffnung und ein Paar Beinöffnungen zu bilden, wobei die Seitenrandzonen (13) des vorderen und des hinteren Hüftbereichs (6, 7) mit daran in der Hüftumfangsrichtung verlaufend angebrachten Befestigungsbändern (30) versehen sind, wobei eine Bandlasche, die jedes der Befestigungsbänder bildet, und eine festgelegte Endzone und eine als Griffstück dienende freie Endzone hat, die in einer Längsrichtung der Bandlasche einander entgegengesetzt sind, wobei die festgelegte Endzone an der Oberfläche des Bekleidungsartikels für den Kontakt mit der Kleidung des Trägers dauerhaft befestigt ist, wobei das Befestigungsband dafür ausgelegt ist, von der festgelegten Endzone zu der freien Endzone hin entfaltet zu werden, wenn das Befestigungsband (30) in der Hüftumfangsrichtung gezogen wird, wobei die freie Endzone (37) zwischen Fingern erfasst ist, und wobei von einer inneren Oberfläche des Befestigungsbandes, welche zu der Oberfläche des Bekleidungsartikel für den Kontakt mit der Kleldung des Trägers weist, wenn das Befestigungsband entfaltet wird, und einer der inneren Oberfläche entgegengesetzten Oberfläche die innere Oberfläche wenigstens teilweise mit einem Befestigungsmittel versehen ist, das an einem bestimmten Teil einer beliebigen der Inneren Oberfläche und der der inneren Oberfläche entgegengesetzten Oberfläche befestigbar ist, welcher Wegwerf-Bekleidungsartikel ferner enthält:
das Bandmaterial (30), welches in einer Z- oder umgekehrten Z-Form gefaltet ist, so dass das Bandmaterial von der festgelegten Endzone zu der freien Endzone hin entfaltet werden kann, welches Befestigungsband ein erstes Bandsegment (31), welches eine oberste Lage der Z- oder umgekehrten Z-Form bildet und die freie Endzone (37) und eine der freien Endzone (37) in der Längsrichtung gegenüberliegende erste Endzone (36) hat, welches erste Bandsegment (31) zwischen den beiden Endzonen (36, 37) auf der inneren Oberfläche mit dem Befestigungsmittel versehen ist, ein zweites Bandsegment (32), welches zwischen dem ersten Bandsegment (31) und der Oberfläche des Bekleldungsartikels (1) für den Kontakt mit der Kleidung des Trägers liegt, so dass es zu der Oberfläche des Bekleidungsartikels für den Kontakt mit der Kleidung des Trägers weist und die festgelegte Endzone (42) und eine der festgelegten Endzone (42) entgegengesetzte zweite Endzone (41) hat, und ein drittes Bandsegment (33) aufweist, welches zwischen dem ersten Bandsegment (31) und dem zweiten Bandsegment (32) liegt und die Bandsegmente (31, 32) miteinander verbindet, welches dritte Bandsegment (33) eine Endzone (46) hat, die mit der ersten Endzone (36) des ersten Bandsegments (31) verbunden ist, und die andere Endzone (47), die der einen Endzone desselben entgegengesetzt ist, mit der zweiten Endzone (42) des zweiten Bandsegments (32) verbunden ist, und eine Zwischenzone (48), die zwischen der einen Endzone (46) und der anderen Endzone (47) gebildet ist und mittels des Befestigungsmittelklebstoffes (39) lösbar an dem ersten Bandsegment (31) befestigt ist, wobei die eine Endzone (46) In der Hüftumfangsrichtung über die festgelegte Endzone (41) des zweiten Bandsegments (33) hinaus verläuft und an der Oberfläche (3) des Bekleidungsartikels (1) für den Kontakt mit der Kleidung des Trägers lösbar befestigt ist, und die äußere Oberfläche der Bandlasche, die in Z- oder umgekehrter Z-Form gefaltet ist, in einem nicht befestigten Zustand in der Nähe einer Region, in welcher die zweite Endzone (42) des zweiten Bandsegments (38) und die andere Endzone (47) des dritten Bandsegments (33) miteinander verbunden sind, sich selbst in nicht befestigtem Zustand gegenüberliegt.

2. Wegwerf-Bekleidungsartikel nach Anspruch 1, bei welchem das Befestigungsmittel (39) ein Klebstoff ist, der auf die innere Oberfläche des ersten Bandsegments (31) aufgetragen ist.

3. Wegwerf-Bekleidungsartikel nach Anspruch 1, bei welchem das Befestigungsmittel ein Hakenteil ist, der an der Innenfläche einer mechanischen Befestigungseinrichtung vorgesehen ist, welche den Hakenteil und einen Schlingenteil umfasst, und der bestimmte Teil mit dem Schlingenteil versehen ist.

4. Wegwerf-Bekleidungsartikel nach Anspruch 3, bei welchem der Schlingenteil ein Vliesstoff ist, welcher eine beliebige der Oberflächen für den Kontakt mit der Haut des Trägers oder der Kleidung des Trägers bildet.

5. Wegwerf-Bekleidungsartikel nach einem der Ansprüche 1 bis 4, bei welchem die erste Endzone (36) des ersten Bandsegments (31) in der Hüftumfangsrichtung über die eine Endzone (46) des dritten Bandsegments (33) hinaus verläuft und an der Oberfläche (3) des Bekleldungsartikels (1) für den Kontakt mit der Kleidung des Trägers lösbar befestigt ist.

6. Wegwerf-Bekleidungsartikel nach einem der Ansprüche 1 bis 5, bei welchem die eine Endzone (46) des dritten Bandsegments (33) sich in der Hüftumfangsrichtung in einem Ausmaß im Bereich von 1 bis 15 mm über die festgelegte Endzone (41) des zweiten Bandsegments (32) hinaus erstreckt.

7. Wegwerf-Bekleidungsartikel einem der Ansprüche 1 bis 6, bei welchem der Bekleidungsartikel ein beliebiger einer Wegwerfwindel des offenen Typs und einer Wegwerfwindel des Höschentyps ist.

## Revendications

1. Article vestimentaire jetable (1) ayant une surface pour contacter un corps de porteur et une surface pour entrer en contact avec les vêtements du porteur, ledit article étant entouré par des zones d'extrémité avant et arrière (11, 12) s'étendant parallèlement entre elles dans une direction de tour de taille et une paire de zones de bordure latérales opposées (13) s'étendant parallèlement entre elles dans une direction longitudinale, ledit article étant composé dans ladite direction longitudinale à ladite direction de tour de taille, d'une région d'entrejambe (8), d'une région avant de taille (6) s'étendant depuis ladite région d'entrejambe (8) à ladite zone d'extrémité avant (11) et d'une région arrière de taille (7) s'étendant depuis ladite région d'entrejambe (8) en direction de ladite zone d'extrémité arrière (12), lesdites régions avant et arrière de taille (6, 7) étant reliées entre elles de manière permanente ou détachable le long des dites zones de bordure latérales (13) de manière à former un trou de taille et une paire de trous de jambe, lesdites zones de bordure latérales (13) de ladite région avant ou arrière de taille (6, 7) étant munies de bandes de fixation (30) attachées à ces dernières de manière à s'étendre dans ladite direction de tour de taille, une attache de bande formant chacune desdites bandes de fixation ayant une zone d'extrémité fixe et une zone d'extrémité libre servant de poignée mutuellement opposée dans une direction longitudinale de l'attache de bande, ladite zone d'extrémité fixe étant attachée de façon permanente à ladite surface dudit article vestimentaire pour entrer en contact avec les vêtements du porteur, ladite bande de fixation étant appropriée pour être dépliée depuis ladite zone d'extrémité fixe en direction de ladite zone d'extrémité libre lorsque ladite bande de fixation (30) est tirée dans ladite direction de tour de taille avec ladite zone d'extrémité libre (37) pincée entre des doigts et, d'une surface interne de ladite bande d'attache faisant face à ladite surface dudit article vestimentaire pour entrer en contact avec les vêtements du porteur lorsque ladite bande de fixation est dépliée et une surface opposée à ladite surface interne, ladite surface Interne étant au moins partiellement munie d'un moyen de fixation attachable à une partie spécifiée dans l'une quelconque de la surface interne et de la surface opposée à ladite surface interne, ledit article vestimentaire jetable comprenant en outre:
ledit matériau de bande (30) étant plié en forme de Z ou de Z inversé de sorte que le matériau de bande peut être déplié depuis ladite zone d'extrémité fixe en direction de ladite zone d'extrémité libre, ladite bande de fixation comprenant un premier segment de bande (31) définissant une couche supérieure de ladite forme en Z ou Z inversé et ayant ladite zone d'extrémité libre (37) et une première zone d'extrémité (36) opposée à ladite zone d'extrémité libre (37) dans ladite direction longitudinale, ledit segment de bande (31) étant muni entre les deux dites zones d'extrémité (36, 37), sur ladite surface interne dudit moyen de fixation, un second segment de bande (32) se trouvant entre ledit premier segment de bande (31) et ladite surface (3) dudit article vestimentaire (1) pour entrer en contact avec les vêtements du porteur de manière à faire face à ladite surface de l'article vestimentaire pour entrer en contact avec lesdits vêtements du porteur et ayant ladite zone d'extrémité fixe (42), et une seconde zone d'extrémité (41) opposée à ladite zone d'extrémité fixe (42) et un troisième segment de bande (33) interposé entre ledit premier segment de bande (31) et ledit second segment de bande (32) et connectant lesdits segments de bande (31, 32) entre eux, ledit troisième segment de bande (33) ayant une zone d'extrémité (46) connectée à ladite première zone d'extrémité (36) dudit premier segment de bande (31) et l'autre zone d'extrémité (47) opposée à ladite zone d'extrémité de cette dernière étant connectée à ladite seconde zone d'extrémité (42) dudit second segment de bande (32) et une zone intermédiaire (48) définie entre ladite zone d'extrémité (46) et ladite autre zone d'extrémité (47) attachée de manière détachable au dit premier segment de bande (31) à l'aide dudit moyen adhésif de fixation (39), ladite zone d'extrémité (46) s'étendant dans ladite direction de tour de taille au-delà de ladite zone d'extrémité fixe (41) dudit second segment de bande (32) et fixée de manière détachable à ladite surface (3) dudit article vestimentaire (1) pour entrer en contact avec lesdits vêtements du porteur, et ladite surface externe de ladite attache de bande pliée en forme de Z ou de Z inversé est opposée à elle-même dans une condition non attachée à proximité d'une région dans laquelle ladite seconde zone d'extrémité (42) dudit second segment de bande (38) et ladite autre zone d'extrémité (47) dudit troisième segment de bande (33) sont connectées entre elles.

2. Article vestimentaire jetable selon la revendication 1, dans lequel ledit moyen de fixation (39) est un adhésif appliqué sur ladite surface interne dudit premier segment de bande (31)

3. Article vestimentaire jetable selon la revendication 1, dans lequel ledit moyen de fixation (39) est une partie de crochet disposée sur ladite surface interne d'une fixation mécanique comprenant une partie en crochet et une partie en boucle et ladite partie spécifiée est munie de ladite partie de boude.

4. Article vestimentaire jetable selon la revendication 3, dans lequel ladite partie de boucle est un tissu non tissé pour former l'une quelconque des surfaces venant en contact avec ladite peau du porteur ou desdits vêtements dudit porteur.

5. Article vestimentaire jetable selon l'une quelconque des revendications 1 à 4, dans lequel ladite première zone d'extrémité (36) dudit premier segment de bande (31) s'étend dans ladite direction de tour de taille au delà d'une dite zone d'extrémité (46) dudit troisième segment de bande (33) et est attachée de manière détachable à ladite surface (3) dudit article vestimentaire (1) entrant en contact avec lesdits vêtements du porteur.

6. Article vestimentaire jetable selon l'une quelconque des revendications 1 à 5, dans lequel ladite zone d'extrémité (46) dudit troisième segment de bande (33) s'étend dans ladite direction de tour de taille au delà de ladite zone d'extrémité fixe (11) dudit second segment de bande (32) dans une dimension comprise entre 1 et 15 mm.

7. Article vestimentaire jetable selon l'une quelconque des revendications 1 à 6, dans lequel ledit article vestimentaire est des couches-culottes jetables du type ouvert et à ceinture élastique.
